Europäisches Patentamt

European Patent Office

Office européen des brevets

(11) Veröffentlichungsnummer : **0 168 782**
**B1**

(12) **EUROPÄISCHE PATENTSCHRIFT**

(45) Veröffentlichungstag der Patentschrift :
17.08.88

(51) Int. Cl.⁴ : **B 01 J 23/66, C 07 D301/10**

(21) Anmeldenummer : 85108667.8

(22) Anmeldetag : 11.07.85

(54) Verfahren zur Verbesserung der Wirksamkeit von Silberträgerkatalysatoren.

(30) Priorität : 20.07.84 DE 3426699

(43) Veröffentlichungstag der Anmeldung :
22.01.86 Patentblatt 86/04

(45) Bekanntmachung des Hinweises auf die Patenterteilung : 17.08.88 Patentblatt 88/33

(84) Benannte Vertragsstaaten :
BE DE FR GB IT NL SE

(56) Entgegenhaltungen :
EP-A- 0 101 008
FR-A- 2 361 155
FR-A- 2 368 298
US-A- 4 350 616

(73) Patentinhaber : HOECHST AKTIENGESELLSCHAFT
Postfach 80 03 20
D-6230 Frankfurt am Main 80 (DE)

(72) Erfinder : Rebsdat, Siegfried, Dr.
Trenbeckstrasse 24
D-8261 Burg, Neuötting 2 (DE)
Erfinder : Mayer, Sigmund
Hochfeilnstrasse 20
D-8269 Burgkirchen (DE)
Erfinder : Alfranseder, Josef
Hauptstrasse 31
D-8261 Hofschallern Post Marktl (DE)
Erfinder : Kaulich, Herbert
Cyprianstrasse 3
D-8262 Altötting (DE)
Erfinder : Schaffelhofer, Iwo, Dr.
Kettelerstrasse 2
D-8263 Burghausen (DE)

**Beschreibung**

Die Erfindung betrifft ein Verfahren zur Verbesserung der Wirksamkeit von Silberträgerkatalysatoren für die Herstellung von Ethylenoxid durch Umsetzung von Ethylen mit Sauerstoff oder Luft.

Zur Herstellung von Ethylenoxid durch Oxidation von Ethylen mit Sauerstoff oder Luft werden Silberträgerkatalysatoren eingesetzt. Eine allgemeine Beschreibung dieser Direktoxidation von Ethylen befindet sich beispielsweise in Kirk-Othmer : Encyclopedia of Chemical Technology, Band 9, Seiten 432 bis 471, John Wiley, London-NY, 1980. Bei dieser Reaktion wird üblicherweise nur ein Bruchteil des eingesetzten Ethylens umgesetzt, wobei Ethylenoxid und als unerwünschte Nebenprodukte im wesentlichen Kohlendioxid und Wasser gebildet werden.

Die Wirksamkeit eines Silberträgerkatalysators wird im wesentlichen durch seine Aktivität und Selektivität bestimmt. Die Selektivität ist der molare Prozentsatz an umgesetztem Ethylen, das zu Ethylenoxid reagiert hat. Die Aktivität wird durch die Ethylenoxid-Konzentration am Reaktorausgang bei sonst konstanten Bedingungen (wie Temperatur, Druck, Gasmenge und Katalysatormenge) charakterisiert. Je höher die Ethylenoxid-Konzentration desto höher ist die Aktivität, oder anders ausgedrückt, je niedriger die zum Erreichen einer bestimmten Ethylenoxid-Konzentration erforderliche Temperatur, desto höher ist die Aktivität. Bei steigenden Rohstoffpreisen und zunehmender Rohstoffverknappung kommt insbesondere einer erhöhten Selektivität der Katalysatoren besondere wirtschaftliche Bedeutung zu.

In den letzten Jahren sind in der Literatur im Prinzip zwei Wege beschrieben worden, mit denen Silberträgerkatalysatoren mit erhöhter Wirksamkeit erhalten werden können. Der eine Weg beruht auf der Entwicklung neuer Katalysatoren. So ist beispielsweise in der US-A-3 962 136 ein Silberträgerkatalysator beschrieben, der dadurch erhalten wird, daß man auf Aluminiumoxid gleichzeitig mit dem Silber einen Promotor aufbringt, und zwar 0,0004 bis 0,0027 g-Äquivalente Kalium, Rubidium und/oder Cäsium pro Kilogramm Katalysator, das sind beispielsweise im Falle von Cäsium 53 bis 360 mg Cäsium pro Kilogramm Katalysator. Der andere Weg zur Herstellung von Silberträgerkatalysatoren mit erhöhter Wirksamkeit beruht darauf, daß bei einem an sich fertigen Katalysator durch eine Nachbehandlung insbesondere die Selektivität verbessert wird. Solche Verfahren sind aus den US-A-4 051 068, US-A-4 123 385, US-A-4 177 169, US-A-4 033 903, US-A-4 186 106, US-A-4 125 480, US-A-4 278 562 und US-A-4 335 014 bekannt.

Bei diesen Verfahren wird der zu behandelnde Katalysator mit einer Lösung, bestehend aus mindestens einer Cäsium- und/oder Rubidiumverbindung und einem Lösungsmittel aus der Gruppe der niedrigen aliphatischen gesättigten Alkohole und Wasser imprägniert (getränkt), um damit etwa 1 bis 1 000 mg Cäsium und/oder Rubidium pro Kilogramm Katalysator als Promotor auf das Trägermaterial (den Silberträgerkatalysator) aufzubrigen, und anschließend die überschüssige Imprägnierlösung (das Lösungsmittel) vom imprägnierten Katalysator abgetrennt. Ein neueres Verfahren dieser Art ist in der US-A-4 478 948 beschrieben. Dabei wird die Behandlung (Imprägnierung) des Katalysators mit der Cäsium- und/oder Rubidiumverbindungen enthaltenden Lösung (Imprägnierlösung) zur Aufbringung von 1 bis 1 000 mg pro Kilogramm Cäsium und/oder Rubidium bei erhöhter Temperatur durchgeführt (Heißimprägnierung).

Was die Cäsium- und Rubidiumverbindungen anbelangt, die bei den bekannten Herstellungsverfahren und Nachbehandlungsverfahren von Silberträgerkatalysatoren eingesetzt werden, wird in allen obengenannten Druckschriften gesagt, daß es für die angestrebte Selektivitätsverbesserung keine Rolle spiele, welcher Art die eingesetzte Cäsium- und Rubidiumverbindung sei ; entscheidend sei lediglich, daß man eine bestimmte Menge an Cäsium und/oder Rubidium, nämlich etwa 1 bis 1 000 mg pro Kilogramm, auf das Trägermaterial aufbringe. Als mögliche Cäsium- und Rubidiumverbindungen werden die verschiedensten anorganischen und organischen Salze sowie Hydroxide und Oxide genannt. Sofern Verbindungen als besonders geeignet oder bevorzugt aufgezählt werden, bezieht sich dies offensichtlich auf rein praktische Gesichtspunkte wie Preis, Zugänglichkeit und Löslichkeit im Lösungsmittel zur Herstellung der Imprägnierlösung.

Es wurde nun überraschenderweise gefunden, daß eine besonders gute Verbesserung der Wirksamkeit bei Silberträgerkatalysatoren erhalten wird, wenn als Imprägnierlösung eine solche eingesetzt wird, die aus einem Cäsiumalkoholat eines niedrigen aliphatischen gesättigten Alkohols oder einer Mischung davon als Cäsiumverbindung und aus einem niedrigen aliphatischen gesättigten Alkohol als Lösungsmittel besteht, und die Nachbehandlung (Imprägnierung) bei erhöhter Temperatur durchgeführt wird.

Das erfindungsgemäße Verfahren zur Verbesserung der Wirksamkeit von Silber-Trägerkatalysatoren für die Herstellung von Ethylenoxid durch Umsetzung von Ethylen mit Sauerstoff oder Luft, wobei auf den zu verbessernden Silber-Trägerkatalysator 1 bis 1 000 mg Cäsium pro kg Katalysator aufgebracht werden, indem der Katalysator mit einer eine Cäsium-Verbindung enthaltenden Lösung bei einer Temperatur von 60 bis 200 °C imprägniert und die überschüssige Imprägnierlösung vom imprägnierten Katalysator abgetrennt wird, ist dadurch gekennzeichnet, daß eine Imprägnierlösung verwendet wird, die das Cäsium im wesentlichen als Cäsiummethylat, Cäsiumethylat, Cäsiumpropylat und/oder Cäsiumisopropylat enthält, und die Imprägnierung bei einer Temperatur von 80 bis 180 °C durchgeführt wird.

Beim erfindungsgemäßen Verfahren wird also eine Imprägnierlösung eingesetzt, die als Cäsiumver-

2

bindung ein Cäsiumalkoholat eines $C_1$- bis $C_3$-Alkohols, das ist Cäsiummethylat, Cäsiumethylat, Cäsiumpropylat oder Cäsiumisopropylat, enthält. Das Lösungsmittel der Imprägnierlösung besteht aus einem $C_1$- bis $C_3$-Alkohol, das ist Methanol, Ethanol, Propanol oder Isopropanol.

Nachdem die erfindungsgemäß einzusetzende Imprägnierlösung Cäsiumalkoholate als Cäsiumverbindungen enthalten soll, sind zur Bereitung der Imprägnierlösung solche Cäsiumverbindungen geeignet, die in dem erfindungsgemäß zu verwendenden Lösungsmittel als Cäsiumalkoholat vorliegen. Solche Cäsiumverbindungen sind Cäsiumhydroxid, Cäsiumalkoholat, Cäsiumoxid und Cäsiummetall, wobei Cäsiumhydroxid und Cäsiumalkoholat bevorzugt sind. Diese Cäsiumverbindungen sind in den genannten Alkoholen löslich. Aus Zweckmäßigkeitsgründen wird der als Lösungsmittel eingesetzte Alkohol mit dem Alkohol im Cäsiumalkoholat übereinstimmen. Beim Cäsiumhydroxid kann es sich um die kristallwasserfreie (CsOH) oder um die kristallwasserhaltige ($CsOH \cdot H_2O$) Verbindung handeln.

Beim erfindungsgemäßen Verfahren beträgt die Imprägniertemperatur vorzugsweise 80 bis 150 °C. Durch das erfindungsgemäße Imprägnieren des zu behandelnden Silberträgerkatalysators sollen 1 bis 1 000 mg Cäsium pro Kilogramm Katalysator, vorzugsweise 50 bis 500 mg Cäsium pro Kilogramm Katalysator, auf den Katalysator aufgebracht werden.

Das Herstellen der Imprägnierlösung im einzelnen, das Imprägnieren des Silberträgerkatalysators und das Abtrennen der überschüssigen Imprägnierlösung vom imprägnierten Katalysator sind an sich bekannte Arbeitsschritte und in den oben angegebenen Druckschriften als solche ausführlich beschrieben.

In der Imprägnierlösung kann die Konzentration an Cäsiumverbindung innerhalb eines weiten Bereiches variieren. Entscheidend ist lediglich, daß mit der erfindungsgemäßen Imprägnierung die oben angegebene Menge an Cäsium, nämlich 1 bis 1 000 mg, vorzugsweise 50 bis 500 mg, Cäsium pro Kilogramm Katalysator auf den in Behandlung stehenden Silberträgerkatalysator niedergeschlagen werden. Die Menge an Imprägnierlösung wird im allgemeinen so gewählt, daß ein volumenmäßiger Überschuß, bezogen auf den zu imprägnierenden Silberträgerkatalysator, vorliegt. In der Regel wird das 0,5-bis 3fache, vorzugsweise 1-bis 2fache Volumen an Imprägnierflüssigkeit, bezogen auf das Volumen an zu imprägnierendem Katalysator genommen.

Die Imprägnierzeit, das ist die Zeit, in der der Katalysator mit der Imprägnierflüssigkeit in Kontakt bleibt, ist klarerweise so zu wählen, daß die erforderliche Menge an Cäsium (Cäsiumverbindung) auf den Katalysator (Träger) aufgebracht wird. Sie hängt vor allem von der Konzentration der Imprägnierlösung an Cäsium (Cäsiumverbindung) und von dem Trägermaterial des zu imprägnierenden Katalysators ab und beträgt allgemein 1 bis 15 Studen, vorzugsweise 3 bis 10 Stunden.

Nach einer zweckmäßigen Methode wird die erfindungsgemäße Imprägnierung im einzelnen in der Weise durchgeführt, daß der Katalysator in einem Behälter (Autoklaven) mit der Imprägnierlösung übergossen wird, Katalysator und Imprägnierflüssigkeit, gegebenenfalls unter Rühren, auf die oben angegebene Temperatur erhitzt und bei dieser Temperatur 1 bis 15 h, vorzugsweise 3 bis 10 h, lang (Kontaktzeit) gehalten werden. Nach einer anderen zweckmäßigen Methode, dem sogenannten Fluten, wird die Imprägnierung an dem in einem oder mehreren Rohren als Festbett angeordneten Katalysator durchgeführt. Diese Methode empfiehlt sich besonders bei Großanlagen, in denen der zu behandelnde Katalysator bereits in den Rohren des Reaktors vorliegt. Das Übergießen (Fluten) kann einmal oder mehrmals (mit der abgetrennten oder mit einer neubereiteten Imprägnierflüssigkeit) kontinuierlich oder diskontinuierlich durchgeführt werden. Beim kontinuierlichen Imprägnieren wird die Imprägnierlösung unter Einhaltung der erforderlichen Kontaktzeit im Kreis gepumpt. Auch im Fall des Flutens ist es zweckmäßig, die Imprägniertemperatur dadurch zu erreichen und einzustellen, daß Katalysator und Imprägnierflüssigkeit auf diese Temperatur erhitzt werden (es versteht sich von selbst, daß bei der erfindungsgemäßen Imprägnierung aufgrund der erhöhten Imprägniertemperatur Drücke auftreten, die den Dampfdrücken der eingesetzten Imprägnierflüssigkeiten entsprechen).

Nach der Imprägnierung wird die überschüssige Imprägnierflüssigkeit vom Katalysator abgetrennt, durch Abgießen, Abfiltrieren, Abzentrifugieren oder im Falle des Flutens einfach durch Abfließenlassen.

Wenn auch der so erhaltene, vom Alkohol und gegebenenfalls von Wasser noch mehr oder weniger feuchte Katalysator beim Aufheizen für den bestimmungsgemäßen Einsatz diese Feuchtigkeit ohnehin verliert, ist es im allgemeinen doch zweckmäßig, nach der erfindungsgemäßen Behandlung eine Trocknung des Katalysators anzuschließen. Diese Trocknung erfolgt im allgemeinen bei einer Temperatur von 20 bis 150 °C, vorzugsweise bei 50 bis 120 °C. Die Abdampfung des Lösungsmittels kann beispielsweise mit Hilfe von heißen Inertgasen wie Luft, Stickstoff und/oder Kohlendioxid beschleunigt werden. Die Temperatur richtet sich im allgemeinen nach dem Siedepunkt des Lösungsmittels der Imprägnierlösung. Nach einer bevorzugten Arbeitsweise wird die Trocknung in Gegenwart von Inertgas und bei einer Temperatur von 50 bis 120 °C durchgeführt.

Nach der Durchführung des erfindungsgemäßen Verfahrens liegt der fertige, in der Wirksamkeit verbesserte Katalysator vor.

Das erfindungsgemäße Verfahren ist unabhängig von der Art des Silberträgerkatalysators. Jeder für die Direktoxidation von Ethylen zu Ethylenoxid mit Sauerstoff oder Luft geeignete Silberträgerkatalysator kommt für das erfindungsgemäße Verfahren in Betracht. Diese Silberkatalysatoren bestehen in der Regel aus Silber in einer Menge von 3 bis 20 Gew.-%, vorzugsweise 7 bis 14 Gew.-%, und gegebenenfalls Promotoren wie Kalium, Natrium, Lithium, Cäsium und/oder Rubidium in einer Menge von 0,002 bis 0,08.

vorzugsweise 0,003 bis 0,05 Gew.-%, auf einem hitzebeständigen, porösen Trägermaterial (Gewichtsprozente jeweils bezogen auf den gesamten Katalysator ; diese Gewichtsprozente entsprechen 20 bis 800 mg pro Kilogramm bzw. 30 bis 500 mg pro Kilogramm). Das Silber befindet sich bekanntlich als Metall auf den inneren und äußeren Oberflächen des Trägermaterials. Die Morphologie des auf dem Trägermaterial aufgebrachten Silbers kann innerhalb weiter Grenzen variieren. Im allgemeinen hat es die Gestalt halbkugelförmiger Teilchen mit einem Durchmesser von 0,01 bis 10 $\mu$m. Beispiele für Träger sind Aluminiumoxide verschiedenster Struktur, Magnesiumoxid, Kieselgur, Bimsstein, Siliciumdioxid, Siliciumcarbid, Tone, Korund, Zeolithe, Metalloxide und ähnliches. Besonders bevorzugte Trägermaterialien sind $\alpha$-Aluminiumoxide, da sie einen weitgehend gleichmäßigen Porendurchmesser aufweisen. Die Trägermaterialien werden in der Regel durch die spezifische Oberfläche (m²/g), das spezifische Porenvolumen (cm³/g) und den mittleren Porendurchmesser ($\mu$m) charakterisiert. Sie weisen im allgemeinen die Form von Granulaten, Kugeln, Ringen oder dergleichen auf.

Wie oben bereits erwähnt, kann jeder für die Direktoxidation von Ethylen zu Ethylenoxid geeignete Silberträgerkatalysator als Ausgangsmaterial beim Verfahren der vorliegenden Erfindung eingesetzt werden. Es kann sich zum Beispiel um einen (promotorhaltigen oder promotorfreien) frisch bereiteten, um einen (promotorhaltigen oder promotorfreien) frisch bereiteten und hitzebehandelten (stabilisierten), um einen mit Promotoren wie Kalium, Natrium, Lithium, Cäsium und/oder Rubidium aktivierten oder um einen gebrauchten Silberträgerkatalysator handeln. In all diesen Fällen wird durch die erfindungsgemäße Behandlung eine Verbesserung der Wirksamkeit erreicht. Unter Hitzebehandlung versteht man bekanntlich das Erhitzen des Katalysators auf erhöhte Temperatur, bis seine Aktivität Konstanz erreicht hat. Unter Gebrauch versteht man bekanntlich, daß der Katalysator zur Umsetzung von Ethylen zu Ethylenoxid mit Sauerstoff oder Luft bereits eingesetzt war. Die Zeit seines Einsatzes kann innerhalb weiter Grenzen variieren ; sie kann wenige Stunden bis mehrere Jahre betragen. Dabei kann der Katalysator in seiner Wirksamkeit nachgelassen oder die ursprüngliche Wirksamkeit im wesentlichen beibehalten haben. Beim erfindungsgemäßen Verfahren werden vorzugsweise solche gebrauchten Silberträgerkatalysatoren eingesetzt, deren Wirksamkeit abgesunken ist, die also zu den sogenannten gealterten oder ermüdeten Silberkatalysatoren zählen. Die gebrauchten Katalysatoren können die genannten Promotoren enthalten oder promotorenfrei sein. Sie können bereits ein- oder mehrmals nach einer der bekannten Methoden regeneriert (reaktiviert) worden sein, beispielsweise nach der Methode der US-A-4 051 068. Das erfindungsgemäße Verfahren ist besonders vorteilhaft im Falle von frisch bereiteten, im Falle von frisch bereiteten und stabilisierten und im Falle von regenerierten Silberträgerkatalysatoren.

Mit dem erfindungsgemäßen Verfahren werden die Aktivität und die Selektivität des Silberträgerkatalysators erhöht. Es ist daher möglich, bei erfindungsgemäß behandelten Katalysatoren die Reaktionstemperatur abzusenken, bei gleichem oder sogar erhöhtem Umsatz. Dies ist deshalb besonders bedeutungsvoll, weil mit niedriger Reaktionstemperatur die Bildung unerwünschter Nebenprodukte wie Kohlendioxid, Formaldehyd und Acetaldehyd stark zurückgeht. Angesichts der großen Mengen Ethylenoxids, die nach dem Ethylen-Oxidationsverfahren produziert werden, kommt einer Ausbeute-Steigerung auch nur um wenige Prozente oder sogar um Zehntel-Prozente erhebliche wirtschaftliche Bedeutung zu.

Die Erfindung wird nun an Beispielen (erfindungsgemäßen Beispielen und Vergleichsbeispielen) noch näher erläutert.

Die Beispiele wurden in einem Versuchsreaktor aus einem senkrecht stehenden Reaktionsrohr aus Chrom-Vanadiumstahl mit einer lichten Weite von 30 mm und einer Länge von 300 mm durchgeführt. Das mit einem Mantel versehene Reaktionsrohr wurde mit heißem Öl, das durch den Mantel strömte, beheizt. Das Reaktionsrohr war bis zu einer Höhe von 200 mm mit $\alpha$-Al$_2$O$_3$-Pellets gefüllt ; diese Füllung diente zur Vorheizung des Einsatzgases. Auf der inerten Füllung lag der zu prüfende Katalysator. Das Einsatzgas trat unten (drucklos) in das Reaktionsrohr ein und verließ es am Kopf. Das eingesetzte Gasgemisch bestand aus :

| | | |
|---|---|---|
| C$_2$H$_4$ | 28 | Vol.-% |
| CH$_4$ | 53 | Vol.-% |
| O$_2$ | 8 | Vol.-% |
| CO$_2$ | 5 | Vol.-% |
| N$_2$ | 6 | Vol.-% |
| Vinylchlorid | 0,0002 Vol.-% | (Inhibitor). |

Die Raum-Zeit-Geschwindigkeit betrug :

$$400 \cdot \frac{\text{Raumteile Gas}}{\text{Stunde} \cdot \text{Raumteile Katalysator}}$$

Die Temperatur des Wärmeträgermediums wurde solange variiert, bis ein konstanter Ethylenumsatz von 7 Vol.-% erzielt wurde.

Das am Reaktorausgang austretende Gas wurde gaschromatographisch analysiert und aus den Analysenergebnissen wurden der Umsatz (die Aktivität) und die Selektivität ermittelt.

4

In den Beispielen wurden die nachstehend angeführten Silberträgerkatalysatoren verwendet :

Katalysator I : ist ein frisch bereiteter Silberträgerkatalysator aus 10 Gew.-% Silber auf α-Aluminium-oxid (spezifische Oberfläche 0,3 m² pro g) und 0,003 Gew.-% Cäsium (das sind 30 mg Cäsium pro kg Katalysator), wobei das Silber und Cäsium in Form von Cäsiumnitrat gleichzeitig auf das Trägermaterial niedergeschlagen worden sind (die Gewichtsprozente beziehen sich auf den Silberträgerkatalysator), der stabilisiert worden ist ; die Stabilisierung (Hitzebehandlung) wurde in der Weise durchgeführt, daß der frisch bereitete Silberträgerkatalysator 10 Stunden lang auf 220 °C gehalten worden ist unter gleichzeitigem Durchleiten eines Gasgemisches aus 95 Mol-% Luft und 5 Mol-% Ethylen.

Katalysator II : ist ein promotorfreier Silberträgerkatalysator aus 11 Gew.-% Silber auf α-Aluminium-oxid (spezifische Oberfläche 0,4 m² pro g), der 3 Jahre lang zur technischen Produktion von Ethylenoxid durch Direktoxidation von Ethylen eingesetzt war, und der nach den 3 Jahren Gebrauch gemäß Beispiel 18 der US-PS-4 051 068 regeneriert worden ist, das heißt, mit einer Lösung aus Methanol als Hauptbestandteil und 0,076 Gew.-% CsOH · H$_2$O (das sind 0,061 Gew.-% Cäsium) bei Raumtemperatur imprägniert worden ist, und anschließend weitere 2 Jahre zur technischen Produktion von Ethylenoxid durch Direktoxidation von Ethylen eingesetzt war.

## Beispiele 1 bis 10

In den Beispielen wurden die Katalysatoren I und II mit Hilfe des beschriebenen Versuchsreaktors unter den angegebenen Bedingungen getestet.

Nachstehend sind Beispiele 1 bis 10 im einzelnen angeführt. Die entscheidenden Bedingungen bei der Durchführung der Beispiele und die Testergebnisse sind in einer Tabelle zusammengefaßt (die nicht erfindungsgemäßen Beispiele sind durch den Zusatz « Vergleichsbeispiel » gekennzeichnet).

## Beispiel 1 (Vergleichsbeispiel)

Der Katalysator I wurde ohne weitere Behandlung getestet.

## Beispiele 2 bis 4 (Vergleichsbeispiele)

Der Katalysator I wurde gemäß US-A- schrift 4 478 948 behandelt und dann getestet, wobei Cäsiumformiat, Cäsiumcarbonat und Cäsiumbicarbonat als Cäsiumverbindungen eingesetzt wurden.

0,937 g Cäsiumformiat wurden in 10 g destilliertem Wasser gelöst, worauf diese Lösung mit Methanol auf 1 000 g Lösung gebracht wurde. Analoge Lösungen wurden mit 0,858 g Cäsiumcarbonat und 1,016 g Cäsiumbicarbonat bereitet. Die drei Imprägnierlösungen bestanden also aus Methanol, 1 Gew.-% Wasser und 0,094 Gew.-% Cäsiumformiat, 0,086 Gew.-% Cäsiumcarbonat bzw. 0,102 Gew.-% Cäsiumbicarbonat, das sind jeweils 0,070 Gew.-% Cäsium. 50 g Katalysator I wurden in einer 250 ml-Edelstahlbombe mit jeweils 40 g Imprägnierlösung übergossen, auf 130 °C erhitzt und 10 h lang bei dieser Temperatur gehalten (Heißimprägnierung mit Cäsiumformiat, Cäsiumcarbonat und Cäsiumbicarbonat als Cäsiumverbindungen). Nach Abkühlen wurde die überschüssige Imprägnierlösung abdekantiert und der feuchte Katalysator jeweils eine Stunde lang im Trockenschrank bei 120 °C getrocknet. Durch die Imprägnierungen wurden jeweils 300 mg Cäsium pro Kilogramm Katalysator aufgebracht.

Der so modifizierte Katalysator II wurde getestet.

## Beispiel 5

Der Katalysator I wurde erfindungsgemäß behandelt und dann getestet, wobei Cäsiummethylat als Cäsiumverbindung eingesetzt wurde.

Es wurde eine Lösung mit 0,863 g Cäsiummethylat und 999,137 g Methanol bereitet. Die Imprägnierlösung bestand also aus Methanol und 0,086 Gew.-% Cäsiummethylat, das sind 0,070 Gew.-% Cäsium. Analog den Beispielen 2 bis 4 wurden 50 g Katalysator I in einer 250 ml-Edelstahlbombe mit 40 g Imprägnierlösung übergossen, auf 130 °C erhitzt und 10 h lang bei dieser Temperatur gehalten. Nach Abkühlen wurde die überschüssige Imprägnierlösung abdekantiert und der feuchte Katalysator eine Stunde lang im Trockenschrank bei 120 °C getrocknet. Durch die Imprägnierung wurden 300 mg Cäsium pro Kilogramm Katalysator aufgebracht.

Der so erfindungsgemäß modifizierte Katalysator I wurde getestet.

## Beispiele 6 bis 10

Mit dem Katalysator II wurde wie bei Katalysator I vorgegangen. Die eingesetzten Cäsiumverbindungen für die Bereitung der Imprägnierlösung, die angewandten Imprägniertemperaturen und die durch die Imprägnierung jeweils aufgebrachte Cäsiummenge in Milligramm Cäsium pro Kilogramm Katalysator sind aus der nachstehenden Tabelle ersichtlich.

Wie die Testergebnisse in der nachstehenden Tabelle zeigen, weisen diejenigen Silberträgerkatalysatoren, die erfindungsgemäß mit Cäsiumalkoholaten als Cäsiumverbindungen heißimprägniert worden sind, überraschenderweise eine bessere Aktivität und Selektivität auf als jene Silberträgerkatalysatoren, die zwar heiß, aber mit anderen Cäsiumverbindungen als den erfindungsgemäß vorgeschlagenen imprägniert worden sind.

Tabelle

| Beispiele (VB = Vergleichs beispiel) | Cäsiumverbin- dung für die Bereitung der Imprä- gnierlösung | Imprägnier- temperatur (°C) | Aufgebrachte Cäsiummenge (mg/kg) | Ergebnis der erfindungsgemäßen Behandlung | |
|---|---|---|---|---|---|
| | | | | Temperatur für 7 % $C_2H_4$-Umsatz (°C) | Selektivität bei 7 % $C_2H_4$-Umsatz (%) |
| Katalysator I | | | | | |
| 1 VB | — | — | — | 206 | 75,1 |
| 2 VB | HCOOCs | 130 | 300 | 197 | 78,0 |
| 3 VB | $Cs_2CO_3$ | 130 | 300 | 193 | 78,2 |
| 4 VB | $CsHCO_3$ | 130 | 300 | 194 | 78,2 |
| 5 | $CH_3OCs$ | 130 | 300 | 191 | 80,6 |
| Katalysator II | | | | | |
| 6 VB | — | — | — | 233 | 73,1 |
| 7 VB | CsCl | 110 | 70 | 230 | 77,5 |
| 8 VB | $CsNO_2$ | 180 | 60 | 229 | 77,8 |
| 9 | $C_2H_5OCs$ | 110 | 70 | 220 | 78,8 |
| 10 | iso-$C_2H_7OCs$ | 180 | 60 | 221 | 78,9 |

**Patentansprüche**

1. Verfahren zur Verbesserung der Wirksamkeit von Silber-Trägerkatalysatoren für die Herstellung von Ethylenoxid durch Umsetzung von Ethylen mit Sauerstoff oder Luft, wobei auf den zu verbessernden Silber-Trägerkatalysator 1 bis 1 000 mg Cäsium pro kg Katalysator aufgebracht werden, indem der Katalysator mit einer eine Cäsium-Verbindung enthaltenen Lösung bei einer Temperatur von 60 bis 200 °C imprägniert und die überschüssige Imprägnierlösung vom imprägnierten Katalysator abgetrennt wird, dadurch gekennzeichnet, daß eine Imprägnierlösung verwendet wird, die das Cäsium im wesentlichen als Cäsiummethylat, Cäsiumethylat, Cäsiumpropylat und/oder Cäsiumisopropylat enthält, und die Imprägnierung bei einer Temperatur von 80 bis 180 °C durchgeführt wird.

2. Verfahren nach Anspruch 1, dadurch gekennzeichnet, daß 50 bis 500 mg Cäsium pro kg Katalysator aufgebracht werden.

**Claims**

1. A process for improving the efficacy of supported silver catalysts for the production of ethylene oxide by reacting ethylene with oxygen or air, in which 1 to 1.000 mg of cesium per kg of catalyst are applied to said catalyst to be improved by impregnating the catalyst with a solution containing a cesium compound at a temperature from 60 to 200 °C and separating the excess of impregnating solution from the impregnated catalyst, characterised in that an impregnating solution is used which contains the cesium essentially as cesium methylate, cesium ethylate, cesium propylate and/or cesium isopropylate and the impregnation is carried out at a temperature from 80 to 180 °C.

2. The process as claimed in claim 1, wherein 50 to 500 mg of cesium per kg of catalyst are applied.

**Revendications**

1. Procédé pour améliorer l'efficacité de catalyseurs supportés à l'argent, destinés à la préparation d'oxyde d'éthylène par réaction de l'éthylène sur l'oxygène ou l'air, 1 à 1 000 mg de césium par kg de catalyseur étant appliqués sur le catalyseur supporté à l'argent à améliorer, le catalyseur étant imprégné à une température de 60 à 200 °C par une solution contenant un composé du césium, et la solution d'imprégnation en excès étant séparée du catalyseur imprégné, caractérisé en ce qu'on utilise une solution d'imprégnation contenant le césium essentiellement sous forme de méthylate de césium, de éthylate de césium, de propylate de césium et/ou d'isopropylate de césium, l'imprégnation étant mise en œuvre à une température de 80 à 180 °C.

2. Procédé selon la revendication 1, caractérisé en ce qu'on applique de 50 à 500 mg de césium par kg de catalyseur.